# EUROPÄISCHE PATENTSCHRIFT

(11) **EP 2 563 243 B1**
(45) Veröffentlichungstag und Bekanntmachung des Hinweises auf die Patenterteilung: **31.07.2019**
(21) Anmeldenummer: 11717606.5
(22) Anmeldetag: 29.04.2011
(51) Int. Cl.: A61B 17/16, A61B 17/34, A61B 10/02, A61B 90/00

(54) **KNOCHENBIOPSIEFRÄSER**
BONE BIOPSY MILL
FRAISE POUR BIOPSIE OSSEUSE

(30) Priorität: 29.04.2010 AT 7102010
(43) Veröffentlichungstag der Anmeldung: 06.03.2013
(73) Patentinhaber: ACMIT Gmbh, 2700 Wiener Neustadt (AT)
(72) Erfinder: WIESER, Mario, 8010 Graz (AT); STOCKER, Peter, 8720 Knittelfeld (AT)
(74) Vertreter: Wirnsberger, Gernot
(86) Internationale Anmeldenummer: PCT/EP2011/056828
(87) Internationale Veröffentlichungsnummer: WO 2011/135070

(56) Entgegenhaltungen:
- EP-A1- 1 943 966
- EP-A1- 2 044 897
- WO-A1-00/45713
- WO-A1-02/13893
- WO-A1-93/08729
- WO-A1-2004/082484
- DE-A1- 3 644 490
- DE-A1-102005 014 624
- DE-B3-102006 045 508
- FR-A1- 2 885 512
- US-A- 4 593 681
- US-A- 4 693 644
- US-A- 4 895 146
- US-A- 5 073 169
- US-A- 5 286 255
- US-A- 5 324 300
- US-A- 5 683 406
- US-A- 5 885 226
- US-A- 6 017 348
- US-A1- 2003 199 879
- US-A1- 2008 287 740
- US-A1- 2010 057 010

## Beschreibung

Die Erfindung betrifft eine Vorrichtung zum Entnehmen eines Knochen- und/oder Knorpelstücks am knöchernen Skelett eines Menschen oder Tieres, insbesondere eines Knochenzylinders, beispielsweise eines Beckenknochenteils eines Menschen.

Des Weiteren betrifft die Erfindung einen Fräser zum Entnehmen eines Knochen- und/oder Knorpelstücks am knöchernen Skelett eines Menschen oder Tieres, insbesondere Knochenzylinders, beispielsweise eines Beckenknochenteils eines Menschen.

Eine Weiterentwicklung von bildgebenden medizinischen Methoden revolutionierte in den letzten Jahrzehnten eine Diagnostik krankheitsbedingter, struktureller Veränderungen von Organen und Geweben. Dies resultierte darin, dass der menschliche Körper für einen Arzt in morphologischer und funktioneller Hinsicht immer transparenter geworden ist. Durch bildgebende medizinische Methoden bietet sich die Möglichkeit, Strukturen und Funktionen des Körpers zu betrachten, ohne dafür ein "anatomisches Fenster" bzw. einen Zugang zur Leibeshöhle schaffen zu müssen. Medizinische diagnostische Verfahren wie z. B. explorative chirurgische Verfahren sind dadurch großteils entbehrlich geworden. Primär dominiert dabei der Vorteil, krankhaftes Gewebe untersuchen zu können, ohne hierfür gesundes Gewebe zerstören zu müssen.

Auch bildgebenden medizinischen Methoden sind Grenzen gesetzt. Speziell histologische Gewebeveränderungen entziehen sich einem Blickfeld radiologischer Methodik. Eine Detektion von neoplastischem Wachstum bzw. die Sicherung einer Diagnose, die nötig ist, um eine adäquate Therapie einzuleiten, muss auch heute noch auf direktem Weg, nämlich im Rahmen einer Biopsie und einer anschließenden histopathologischen Auswertung, erfolgen.

Im Laufe der Zeit haben sich verschiedene Biopsietechniken etabliert. Dabei erfordert jedes Gewebe, bedingt durch eine individuelle Struktur und materielle Beschaffenheit sowie Konsistenz, spezifisch ausgerichtete Instrumente. Grundsätzlich sollte ein Zugriff auf das zu biopsierende Gewebe möglichst schonend und im sicheren Abstand zu anderen Organsystemen erfolgen. Oft müssen mehrere Schichten von Bindegewebe, Fettgewebe, Haut und Muskulatur durchsetzt werden, um eine gewünschte Gewebeprobe entnehmen zu können - mit Ausnahme der Haut, die für Probeentnahmen leicht zugänglich ist. Prinzipiell kann jedem Organ Parenchym entnommen werden, wofür meist Spezialkanülen in Nadelkonfiguration oder spezielle Stanzen für die Entnahme von Gewebezylindern zum Einsatz kommen. In den meisten medizinischen Teilbereichen haben sich infolgedessen sehr unterschiedliche Biopsietechniken etabliert, beispielsweise in den Gebieten der Dermatologie, Urologie oder Neurochirurgie.

Knochengewebe nimmt vergleichsweise durch seine materielle Eigenheit eine Sonderstellung ein. Rein zytologische Analyseverfahren erfordern keine speziellen Geräte, sondern kommen mit herkömmlichen Nadeln (sogenannte "Jamshidi"-Nadeln) aus. Kompressionsfrakturen im Knochenaufbau haben keine Folgen. Eine strukturelle Auswertung von Knochenproben, z. B. im Rahmen der osteologischen Basisdiagnostik, oder eine Entnahme und Verwendung von Knochen- und/oder Knorpel-Transplantaten bedarf aber einer vollständigen Erhaltung einer in-vivo bestehenden Knochenkonstitution. Ein Knochenzylinder muss, ohne dabei Druck auf die Knochenstruktur auszuüben, aus dessen Verband gesägt oder gefräst werden. Dabei hat die Erhaltung der strukturellen Gegebenheiten oberste Priorität.

Bisher verwendete osteologische Biopsiewerkzeuge bzw. osteochirurgische Werkzeuge können die qualitativen Anforderungen bezüglich eines erforderlichen Grades an Invasivität und eines Erhalts der Knochenstruktur eines Knochen- bzw. Knorpel-KnochenZylinders nicht vollständig erfüllen. Darüber hinaus sind die bekannten Werkzeuge kompliziert zu betätigen, was eine Gewebeentnahme erschwert und einen Patienten unnötig belastet.

Aufgabe der Erfindung ist es, eine Vorrichtung der eingangs genannten Art anzugeben, welche diese Nachteile nicht aufweist.

Eine weitere Aufgabe der Erfindung ist es, einen Fräser der eingangs genannten Art anzugeben, mit dem unter Erhaltung einer in-vivo bestehenden Knochen- und/oder Knorpelstruktur ohne Verletzung eines unter dem Knochen liegendem Gewebes ein Knochen- und/oder Knorpelstück wie ein Knochenzylinder entnommen werden kann.

Die genannte Aufgabe wird bei einer Vorrichtung der eingangs genannten Art dadurch gelöst, dass ein hohler Gewebekanal und ein hohler Fräser mit endseitigen Schneiden vorgesehen sind, wobei der Fräser im Inneren des Gewebekanals geführt ist.

Ein mit der Erfindung erzielter Vorteil ist insbesondere darin zu sehen, dass ein einfach aufgebautes Werkzeug bereitgestellt ist, mit welchem ein Knochen- und/oder Knorpelstück unter Aufrechterhaltung einer In-vivo-Struktur eines Knochens und/oder Knorpels entnommen werden kann. Dabei sorgt der vorgesehene Gewebekanal zum einen für eine stabile Führung des hohlen Fräsers, was erforderlich ist, um ein intaktes Knochen- und/oder Knochenstück wie einen Knochenzylinder entnehmen zu können. Zum anderen sorgt der Gewebekanal dafür, dass die Haut und darunterliegendes Gewebe während der Entnahme des Knochen- und/oder Knorpelstücks nur minimalen Belastungen ausgesetzt sind.

Der Gewebekanal ist erfindungsgemäss so ausgebildet, dass dieser endseitig durch einen zulaufenden Konus bildende Klappen verschlossen ist. Der Gewebekanal kann dann problemlos im Bereich einer Balkenmarkierung, die eine Faserrichtung kennzeichnet, in einen Körper eingeführt werden. In diesem Zusammenhang ist es vorteilhaft, dass ein hohler Arbeitskanal vorgesehen ist, der im Gewebekanal innen anliegend angeordnet ist und mit dem durch Vorschub die Klappen öffenbar sind, wobei der Fräser im Arbeitskanal geführt ist. Der Arbeitskanal ist in diesem Fall relativ zum Gewebekanal in diesem bewegbar, sodass sich durch Vorschub des Arbeitskanals die Klappen des Gewebekanals öffnen lassen. Dies ist zweckmäßig, wenn der Gewebekanal bereits in den Körper eingeführt ist, sodass durch Öffnen der Klappen der Gewebekanal im Körper, speziell am Periost bzw. Knochen, verankerbar ist.

Der Gewebekanal besteht bevorzugt aus Kunststoff und ist insbesondere einteilig ausgebildet. Beispielsweise kann der Gewebekanal als Spritzgussteil hergestellt sein. Dies ermöglicht eine kostengünstige Herstellung des Gewebekanals. Grundsätzlich ist es aber auch denkbar, dass der Gewebekanal aus einem Metall oder einer Legierung besteht.

Zweckmäßig ist es, dass zwei Klappen vorgesehen sind. Durch die zwei Klappen, die nach Einführen des Gewebekanals in einen Körper zur Seite hin geöffnet werden, ist eine ausreichende Fixierung des Gewebekanals im Körper gegeben. Möglich ist es aber auch, eine andere Zahl von Klappen vorzusehen, beispielsweise vier oder mehr, sodass der Gewebekanal endseitig nach Öffnen der Klappen spinnenartig im Gewebe verankerbar ist.

Damit der Gewebekanal nach Einführen in einen Körper besonders gut nach Öffnen der Klappen verankerbar ist, ist vorteilhafterweise vorgesehen, dass die Klappen an deren Enden zumindest teilweise, vorzugsweise jeweils, ein oder mehrere Schneidelemente wie Metallklingen oder allgemein Verankerungselemente aufweisen. Schneidelemente erlauben auch ein Einführen des Gewebekanals in einen Körper ohne vorherigen Schnitt und erfüllen damit eine duale Funktion.

Insbesondere wenn der Gewebekanal aus einem Kunststoff besteht oder zumindest teilweise aus diesem gebildet ist, sind die Klappen vor dem Öffnen über Sollbruchstellen miteinander verbunden. Eine axiale Länge der Klappen in Richtung des Gewebekanals beträgt vorzugsweise weniger als 30 mm, bevorzugt 18 bis 28 mm. Eine derartige optimale axiale Erstreckung der Klappen wurde in einer Vielzahl von Versuchen ermittelt und steht unter anderem in direktem Zusammenhang mit einem auf den Klappen lastenden Weichteildruck und einer Beschaffenheit des verwendeten Kunststoffs.

Einer einfachen Hantierbarkeit wegen ist es vorteilhaft, wenn der Gewebekanal mit einem endseitigen Griffstück ausgebildet ist und der Arbeitskanal einen endseitig lösbar befestigten Stempel aufweist, der in Vorschubrichtung nicht über das Griffstück hinausbewegbar ist. Ein Schaft des Arbeitskanals endet am Stempel und überragt den Gewebekanal aus funktionellen Gründen in seiner Länge. Der Stempel, der bevorzugt aus einem Kunststoff besteht, ist am Schaft des Arbeitskanals wie eine Verschlussklappe aufgesetzt und dient als Auflagefläche bei einer manuellen Kraftübertragung bei Einführen des Arbeitskanals durch Daumendruck. In diesem Zusammenhang ist es auch zweckmäßig, dass ein auf den Gewebekanal aufsetzbares Distanzstück vorgesehen ist, über das der Stempel des Arbeitskanals in Vorschubrichtung nicht hinausbewegbar ist. Dieses Distanzstück wird bei Einführen des Gewebekanals und des anliegenden Arbeitskanals in das Gewebe benötigt. Während dieser Phase sollen die Klappen geschlossen sein und durch deren zulaufenden Konus bzw. die endseitigen Schneidelemente wie Metallklingen ein leichtes Einführen des Gewebekanals samt Arbeitskanal ermöglichen. Durch das Distanzstück ist sichergestellt, dass der Arbeitskanal während dieser Phase nicht über ein vorbestimmtes Maß in den Gewebekanal vorschiebbar ist. In jedem Fall ist eine Länge des Distanzstücks so gewählt, dass während dieser Phase die Klappen nicht durch den Arbeitskanal öffenbar sind.

Der Arbeitskanal besteht vorzugsweise aus einem Metall oder einer Legierung, insbesondere einem Chromstahl. Ein Chromstahl hat sich im Hinblick auf eine hohe Korrosionsbeständigkeit sowie eine Führung des Fräsers als zweckmäßig erwiesen. Der Stempel kann ebenfalls aus einem Metall oder einer Legierung bestehen, bevorzugt ist diesbezüglich jedoch ein Kunststoffelement als Stempel vorgesehen.

Der Arbeitskanal ist des Weiteren bevorzugt an einem den Klappen zugewendeten Ende mit Zacken ausgebildet. Dadurch ist es möglich, nachdem der Gewebekanal und der Arbeitskanal in ein Gewebe eingebracht und die Klappen geöffnet sind, dass der Arbeitskanal ebenfalls im Gewebe, speziell am Periost bzw. Knochen oder Knorpel verankert wird.

Der Gewebekanal ist mit Vorteil mit seitlichen Sicherungsrasten ausgebildet, die über den Klappen angeordnet sind, damit der Gewebekanal relativ zu einer Einrichtung zur anatomischen Positionierung desselben fixierbar ist.

Der vorgesehene Fräser weist zweckmäßigerweise einen endseitig in Vorschubrichtung im Durchmesser verbreiterten Fräskopf mit abwechselnd angeordneten Hauptschneiden und Vorschneiden auf. Durch den größeren Durchmesser des Fräskopfes entsteht ein geringfügiger Spalt bzw. eine Freistellung zwischen dem Fräser und dem Arbeitskanal. In diesen Spalt werden während einer Schneidoperation bzw. der Entnahme eines Knochen- und/oder Knorpelstücks, z. B. eines Knochenbiopsats, Späne abgeführt. Ist ein Knochen- und/oder Knorpelstück entnommen, so kann der Fräser aus dem Arbeitskanal gezogen werden, wobei aufgrund der im Querschnitt konischen Verbreiterung des Fräskopfes die Späne automatisch mit entfernt werden. Somit ist auf einfache Weise sichergestellt, dass die abgespanten Späne gesichert und danach auf einfache Weise entfernt werden. Eine Ausbildung mit Hauptschneiden und Vorschneiden ist zweckmäßig, da die in der Regel die Hauptschneiden überragenden Vorschneiden formgebend auf einen zu entnehmenden Knochen- bzw. Knochen-Knorpel-Zylinder wirken, wohingegen die Hauptschneiden den größeren Anteil der Zerspanungsarbeit übernehmen und für genügend Freistellung in der Schnittfuge am Außendurchmesser des Fräsers sorgen. Darüber hinaus kann durch eine abwechselnde Verteilung von Vorschneiden und Hauptschneiden ein effektiver Druck pro Schneide verringert werden, was wiederum Kompressionsfrakturen der Spongiosabälkchen verhindert. Ferner ist ein Verschleiß an den Schneiden reduziert, was sich in weiterer Folge positiv auf eine Standzeit des Werkzeuges auswirkt. Eine hohe Qualität einer Knochenprobe kann dadurch über eine längere Zeit mit dem gleichen Fräser gewährleistet werden.

Der Fräser ist bevorzugt aus einem Stahl gebildet, da dieser im Einsatz den größten Belastungen der Teile einer erfindungsgemäßen Vorrichtung unterliegt. Um eine Standzeit zu erhöhen, kann diesbezüglich auch vorgesehen sein, dass der Fräser zumindest im Bereich der Hauptschneiden und Vorschneiden beschichtet ist.

Besonders günstig hat es sich für die Entnahme eines Knochen-und/oder Knorpelstücks erwiesen, wenn im Fräser eine innen anliegende hohle Eprouvette positioniert ist. In dieser Eprouvette kann ein entnommenes Knochen- und/oder Knorpelstück, z. B. ein Knochenzylinder, gesichert werden. Für diese Zwecke ist die Eprouvette relativ zum Fräser fixiert.

Die Eprouvette ist keinen besonderen mechanischen Belastungen ausgesetzt und soll das zu entnehmende bzw. entnommene Knochenbiopsat nicht verletzen. Deshalb besteht die Eprouvette bevorzugt aus einem Kunststoff wie Polyethylen oder dergleichen.

Die Eprouvette ist im Bereich eines ersten Endes, das benachbart zum Fräskopf liegt, durch eine Membran mit einem Schlitz verschlossen. Der Schlitz ist dabei zentral angebracht. Dadurch fungiert die Membran als Greifwerkzeug, indem diese den aus dem Knochenverband gelösten Gewebezylinder bzw. den Knochen- und/oder Knorpelzylinder wie ein Knochenbiopsat oder einen Knochenzylinder zum Transplantieren am Umfang umschließt. Durch eine fortlaufende Arbeitsbewegung wird der Gewebezylinder immer weiter in den Schaft des Fräsers geführt, direkt in den zentralen Schlitz der Membran, und in weiterer Folge in die Eprouvette. Die Eprouvette kann nach Abschluss des Vorgangs durch Lösen einer Fixierung samt dem Gewebezylinder bzw. dem Knochen- und/oder Knorpelzylinder aus dem Fräser gezogen werden. Im Bereich eines zweiten Endes ist die Eprouvette durch ein Griffteil verschlossen, wobei Sollbruchstellen vorgesehen sind, sodass das Griffteil von der Eprouvette abbrechbar ist. Das Griffteil dient einer leichten Entnehmbarkeit der Eprouvette aus dem Fräser. Da das Griffteil abgebrochen oder bei einer lösbaren Anordnung, z. B. durch Verschrauben, auch ohne Brechen abgenommen werden kann, braucht in der Folge die Eprouvette lediglich umgedreht zu werden, um den Gewebezylinder bzw. das Knochen- und/oder Knorpelstück entnehmen zu können.

Der Fräser ist mit einem hohlen Schaft ausgebildet und weist bevorzugt endseitig einen Fräskopf mit abwechselnd angeordneten Hauptschneiden und Vorschneiden auf, wobei die Vorschneiden die Hauptschneiden in axialer Richtung überragen, und/oder am Fräser sind neben vertikal schneidenden Schneiden zusätzliche Schneiden vorgesehen, die normal zu einer Längsachse des Fräsers schneiden und bei Bedarf aktivierbar sind. Ein Vorteil eines erfindungsgemäßen Fräsers ist darin zu sehen, dass mit diesem ein Knochen- und/oder Knorpelstück wie ein Knochenbiopsat oder Knochentransplantat unter Erhaltung der bestehenden In-vivo-Knochenstruktur aus einem Körper entnommen werden kann. Hauptschneiden übernehmen den größeren Anteil des Spanvolumens und weisen bevorzugt einen größeren Spitzenwinkel als die Vorschneiden auf. Infolge einer Längendifferenz bzw. einem Vorragen der Vorschneiden über die Hauptschneiden und einem insbesondere kleineren Spitzenwinkel dienen die Vorschneiden einem verlaufsfreien Anschnitt der Knochenoberfläche. Eine Mehrzahl von Vorschneiden sorgt in weiterer Folge, zusammen mit einem Arbeitskanal, für eine exakte Führung des Fräsers im Knochen- bzw. Knorpel-Knochengewebe. Die Vorschneiden durchdringen dabei eine gesamte Stärke eines Knochens, noch ehe die Hauptschneiden diesen durchdringen, sodass das Knochen- und/oder Knorpelstück wie ein Knochenzylinder aus dem Gewebeverband gelöst wird, bevor die Hauptschneiden den Knochen vollständig durchsetzen. Alternativ oder auch zusätzlich können axial wirkende Schneiden vorgesehen sein, die ein Absetzen eines Knochen- und/oder Knorpelstücks erlauben, ehe eine Schneide einen Knochen und/oder Knorpel durchsetzt hat.

Bevorzugt ist vorgesehen, dass Hauptschneiden und Vorschneiden vorgesehen sind und die Vorschneiden die Hauptschneiden um etwa 0,25 bis 0,75 mm überragen.

Besonders bevorzugt ist vorgesehen, dass der Fräskopf zum Ende hin bzw. in Fräsrichtung einen größeren Durchmesser als der hohle Schaft aufweist. In diesem Zusammenhang ist es auch zweckmäßig, dass am Fräskopf Spannuten vorgesehen sind. Wird der Fräser zusammen mit einem Arbeitskanal eingesetzt, so können abgenommene Späne über die Spannuten in einen Spalt zwischen dem Schaft des Fräsers und dem Arbeitskanal abtransportiert werden.

Insbesondere kann es gewünscht sein, dass ein Knochen- und/oder Knorpelstück entnommen wird, ohne dass ein Knochen vollständig durchfräst wird. In diesem Fall ist es notwendig, das Knochen- und/oder Knorpelstück in einer bestimmten Eindringtiefe vom bearbeiteten Knochen abzusetzen. Zu diesem Zweck sind die erwähnten zusätzlichen Schneiden vorgesehen, die normal zu einer Längsachse des Fräsers schneiden und bei Bedarf aktivierbar sind. Diese Schneiden werden bei Erreichen einer vorbestimmten Eindringtiefe aktiviert, sind bis dahin jedoch inaktiv. Beispielsweise kann vorgesehen sein, dass die zusätzlichen Schneiden durch Magnete in einer umfangsseitigen inaktiven Position gehalten sind und bei Aktivierung zur Längsachse hin schneiden.

Die Erfindung kann mit einer Einrichtung zur erleichterten anatomischen Positionierung einer erfindungsgemäßen Vorrichtung an einem menschlichen oder tierischen Körper verwendet werden, insbesondere im Bereich der Regio glutaea, wobei eine Druckplatte mit einem Rahmen und zumindest einer Öffnung zum Einführen der Vorrichtung und eine Haftfolie zum Befestigen der Einrichtung am Körper vorgesehen sind, wobei die Druckplatte von der Haftfolie umgeben ist und die Druckplatte handbetätigt in eine gewölbte Stellung bringbar ist.

Diese Einrichtung hat die Vorteile, dass einerseits ein Zentriersystem für die Vorrichtung zur Entnahme des Knochen- und/oder Knochenstücks wie eines Knochenzylinders, z. B. eines Knochenbiopsats, geschaffen ist und andererseits sogleich eine 3-Stufen-Wundversorgungseinheit zur Verfügung gestellt wird. Mit der Druckplatte, die von einer Haftfolie umgeben ist, kann durch Betätigen bzw. Auswölben ein Druck auf das Gewebe ausgeübt werden. Dadurch können allfällige Blutungen hintangehalten werden. Darüber hinaus ist die Druckplatte dann für die spätere Positionierung der Vorrichtung positionsstabil gehalten. Die Haftfolie wiederum sorgt dafür, dass die Druckplatte entsprechend in Position gehalten wird. Darüber hinaus dient die Haftfolie während eines Eingriffs als Keimbarriere.

Bevorzugt ist vorgesehen, dass in der zumindest einen Öffnung eine von der Druckplatte lösbare Membran positioniert ist, die eine balkenförmige Markierung aufweist. Die balkenförmige Markierung dient einer Verdeutlichung einer Faserverlaufsrichtung von muskulären und bindegewebigen Strukturen, die für eine Gewebeentnahme mit dem Gewebekanal bzw. dessen Schneidelementen wie Metallklingen durchsetzt werden müssen. Im Anschluss an den Eingriff kann die Haftfolie und auch die Druckplatte von der Membran gelöst werden, die nach wie vor im Bereich eines notwendigen Schnittes im Gewebe anliegt und dann zur Wundrandstabilisation, Wundrandadaptierung und zum Wundverschluss dient.

Die Druckplatte und der Rahmen sind bevorzugt einteilig aus einem Kunststoff gefertigt, insbesondere im Spritzgussverfahren.

Die Haftfolie weist mit Vorteil mehrere flügelartige Laschen auf, was eine feste Anbringung der Einrichtung in verschiedenen Bereichen an der Hautoberfläche eines Körpers ermöglicht.

Zur späteren Wundversorgung ist vorgesehen, dass die Haftfolie am Rahmen mit der Druckplatte verbunden ist und Sollbruchstellen für eine Trennung der Haftfolie vom Rahmen vorgesehen sind. Dadurch kann nach einem Eingriff in einem ersten Schritt die Haftfolie abgenommen werden, ohne dass noch die Druckplatte gelöst werden muss, die zur Wund- bzw. Gefäßkompression dient.

Wenn die Einrichtung mit einer erfindungsgemäßen Vorrichtung zusammenwirkt, ist es von Vorteil, dass der Rahmen eine Nut aufweist, in welche ein Griffstück des Gewebekanals zur temporären Fixierung einführbar ist. Dadurch kann der Gewebekanal in der Einrichtung bzw. am Rahmen der Druckplatte fest fixiert werden, was für ein späteres Einführen eines Fräsers und dessen positionsstabile Führung während einer Knochen- und/oder Knorpelentnahme von Vorteil ist.

Weitere Merkmale, Vorteile und Wirkungen der Erfindung ergeben sich aus den nachfolgenden Ausführungsbeispielen. In den Zeichnungen, auf welche dabei Bezug genommen wird, zeigen:
Fig. 1 eine perspektivische Darstellung eines Gewebekanals;
Fig. 2 eine stirnseitige Ansicht eines Gewebekanals;
Fig. 3 eine Seitenansicht eines Gewebekanals;
Fig. 4 eine perspektivische Darstellung eines Arbeitskanals;
Fig. 5 eine Seitenansicht eines Arbeitskanals;
Fig. 6 eine perspektivische Darstellung eines Distanzstücks;
Fig. 7 einen Teil eines Fräsers im Bereich eines Fräskopfes im Querschnitt;
Fig. 8 eine Seitenansicht eines Fräsers im Bereich eines Fräskopfes;
Fig. 9 eine schematische Darstellung der Schneidverhältnisse an einem Fräskopf;
Fig. 10 eine weitere Darstellung entsprechend Fig. 9;
Fig. 11 einen Schnitt entlang der Linie XI-XI in Fig. 9 bzw. 10;
Fig. 12 einen Querschnitt durch einen Fräser im Bereich axialer Schneiden;
Fig. 13 eine Darstellung gemäß Fig. 12, wobei die Schneiden aktiviert sind;
Fig. 14 eine schematische Darstellung eines Aktivierungsmechanismus axialer Schneiden;
Fig. 15 eine Eprouvette in perspektivischer Darstellung;
Fig. 16 eine Eprouvette mit abgebrochenem Griffteil;
Fig. 17 eine erfindungsgemäße Vorrichtung in einer ersten Arbeitsposition;
Fig. 18 eine erfindungsgemäße Vorrichtung in einer zweiten Arbeitsposition;
Fig. 19 eine Druckplatte in Draufsicht;
Fig. 20 einen Schnitt entlang der Linie XX-XX in Fig. 19;
Fig. 21 eine stirnseitige Ansicht der Druckplatte gemäß Fig. 19;
Fig. 22 eine Ansicht einer Druckplatte gemäß Fig. 19 von unten;
Fig. 23 eine perspektivische Darstellung der Druckplatte gemäß Fig. 22 von unten;
Fig. 24 eine Haftfolie;
Fig. 25 eine schematische Darstellung einer Fotografie bei einer Einführung eines Gewebekanals samt Arbeitskanal in einen menschlichen Körper;
Fig. 26 eine schematische Darstellung einer Fotografie eines Gewebekanals mit darin angeordnetem Arbeitskanal beim Öffnen der Klappen;
Fig. 27 eine schematische Darstellung einer Fotografie einer Haftfolie mit einer Druckplatte sowie in der Druckplatte fixiertem Gewebekanal;
Fig. 28 eine schematische Darstellung einer Fotografie eines Beckenknochens, aus dem zwei Knochenbiopsate entnommen wurden;
Fig. 29 eine schematische Darstellung von aus einem Beckenknochen entnommenen Knochenbiopsaten.

Im Folgenden sind die einzelnen Teile einer erfindungsgemäßen Vorrichtung 1 einzeln näher dargestellt, wonach deren Kombination und Anwendung beschrieben ist.

### Gewebekanal

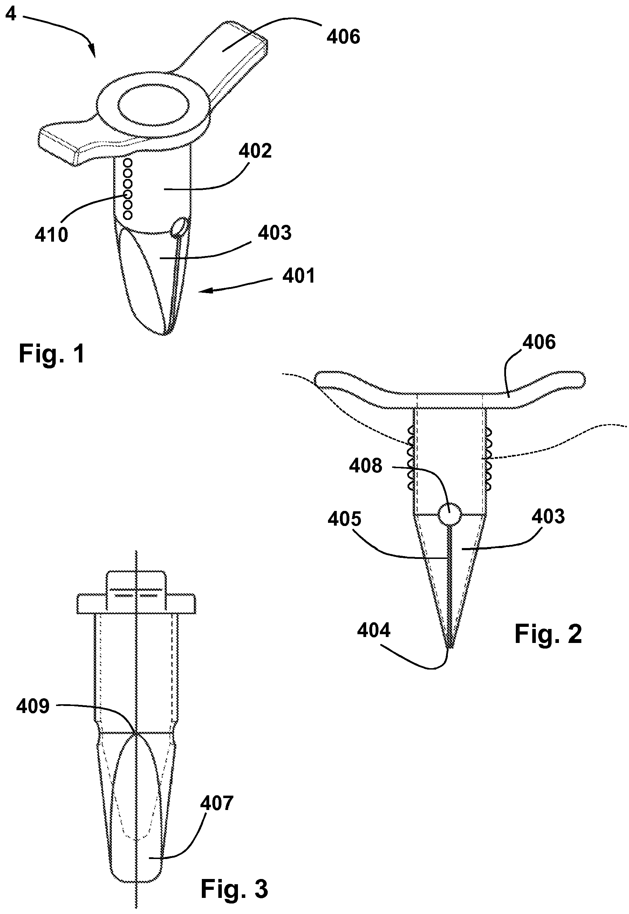

In Fig. 1 bis 3 ist ein Gewebekanal 4, der Teil einer erfindungsgemäßen Vorrichtung 1 ist, näher dargestellt. Der Gewebekanal 4 ist aus einem Kunststoff, beispielsweise Polyethylen oder Polypropylen, gefertigt. Bevorzugt ist es, dass der Gewebekanal 4 einteilig im Spritzgussverfahren erstellt ist. Der Gewebekanal 4 umfasst einen Grundkörper 402, der im Wesentlichen als hohler, im Querschnitt kreisförmiger Zylinder ausgebildet ist. Der zylindrische Grundkörper 402 bildet eine äußerste von insgesamt vier Schichten der Vorrichtung 1, was später noch näher erläutert wird. In einer Arbeitsposition grenzt der Grundkörper 402 mit seiner Umfangsfläche direkt an umgebene Weichteile an. Der Grundkörper 402 bildet somit einen abgegrenzten und gesicherten Durchgang, in dem einzelne Schritte einer Knochen- und/oder Knorpelentnahme bzw. eines Biopsievorganges durchgeführt werden können.

Eine geometrische Form des Grundkörpers 402 beschreibt einen Hohlzylinder mit einem Außendurchmesser von etwa 12,0 bis 14,0 mm und einer lichten Weite von etwa 12,5 mm. Es versteht sich, dass diese Maße je nach Anwendungsfall auch abgewandelt werden können. Eine daraus resultierende Wandstärke liegt z. B. etwa bei 0,5 mm und erstreckt sich konstant über eine Gesamtlänge des Grundkörpers 402. An einem Ende des Grundkörpers 402 ist ein seitlich auskragendes Griffstück 406 vorgesehen. Das Griffstück 406 umfasst zwei in einem vorgegebenen Winkel nach oben auskragende Seitenteile. Gemäß Fig. 1 sind zwei Seitenteile vorgesehen, die in einem Winkel von 180° zueinander angeordnet sind. Gleichwohl können auch mehr Seitenteile vorgesehen sein, wenngleich sich zwei Seitenteile für ein Hantieren als zweckmäßig erwiesen haben. An einem anderen Ende des Grundkörpers 402 schließen Klappen 403 an, die einen Konus 401 bilden. In Fig. 1 bis 3 sind zwei Klappen 403 dargestellt, wenngleich auch mehr als zwei Klappen 403 vorgesehen sein können, beispielsweise vier oder mehr Klappen 403.

Eine Länge des Grundkörpers 402 wurde in zahlreichen Versuchen ermittelt und steht in direktem Zusammenhang mit einem auf den Klappen 403 lastenden Weichteildruck, einem Durchmesser des Grundkörpers 402, einer geforderten lichten Weite und einer Beschaffenheit des verwendeten Kunststoffs. Grundsätzlich liegt eine axiale Erstreckung des Grundkörpers 402 unterhalb von 30 mm.

Eine ermittelte axiale Länge der Klappen 403 beträgt bevorzugt etwa 25 mm und ist konstant, auch bei Änderung einer Gesamtlänge des Gewebekanals 4.

Die Klappen 403 sind in axialer Richtung durch Sollbruchstellen 405 miteinander verbunden. Des Weiteren ist eine Ausnehmung 408 vorgesehen, sodass die Klappen 403 nach Durchtrennen der Sollbruchstellen 405 auf einfache Weise um einen Schwenkpunkt 409 nach außen geschwenkt werden können. Diesbezüglich weisen die Klappen 403 bevorzugt abgeschrägte Flächen 407 auf, sodass eine Schwenkbarkeit um einen einzelnen Schwenkpunkt 409 möglich ist. Die abgeschrägten Flächen 407 ermöglichen überdies ein relativ leichtes Einführen des Gewebekanals 4 in einen Körper. Endseitig sind die Klappen 403 mit Schneidelementen 404 bzw. Metallklingen aus einem Metall oder einer Legierung bestückt.

### Arbeitskanal

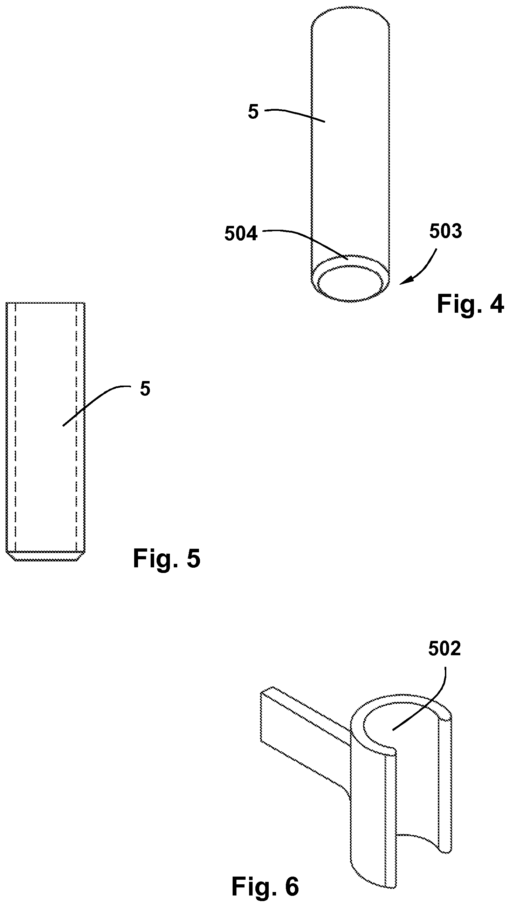

In Fig. 4 und 5 ist ein Arbeitskanal 5 dargestellt. Der Arbeitskanal 5 ist ein hohler zylindrischer Körper, der bevorzugt aus einem Edelstahl, z. B. einem Chromstahl, gebildet ist. Der Arbeitskanal 5 ist an beiden Enden offen und weist an einem Ende 503 in Fig. 4 bloß angedeutete Zacken 504 auf. Eine Wandstärke des Arbeitskanals 5 beträgt beispielsweise 1,1 mm bei einem Durchmesser von 12 mm und 9,8 mm lichter Weite. Ein äußerer Durchmesser des Arbeitskanals 5 entspricht einem inneren Durchmesser des Gewebekanals 4 im Bereich des Grundkörpers 402.

Mit dem Arbeitskanal 5 wirkt im später erläuterten Einsatz ein Distanzstück 502 zusammen, das auf den Gewebekanal 4 stülpbar ist. Um die dafür erforderliche Flexibilität zu gewährleisten, ist das Distanzstück 502 aus einem länglichen, im Querschnitt zur Längsachse u-förmig ausgebildeten Kunststoffteil gebildet. An dem Kunststoffteil ist zur einfachen Hantierbarkeit integral ein abstehendes Halteelement angeformt.

### Fräser

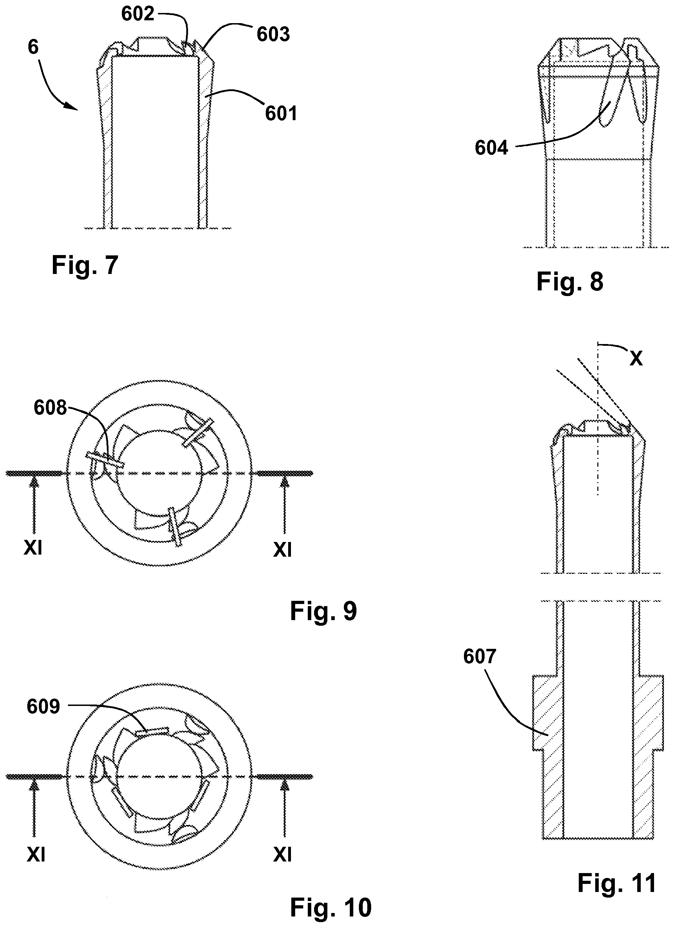

In Fig. 7 bis 14 ist ein erfindungsgemäßer Fräser 6 bzw. Teile davon näher dargestellt. Der Fräser 6 weist einen hohlen, zylindrischen Fräserschaft auf. An einem Ende des Fräserschaftes ist ein Einspannteil 607 integral angeformt. Das Einspannteil 607 dient einer Einspannung des Fräsers 6. An dem anderen Ende des Fräserschaftes ist ebenfalls integral ein Fräskopf 601 angeformt. Der Fräskopf 601 ist vom Fräserschaft aus betrachtet zum Ende hin konisch verbreitert bzw. im Querschnitt mit verbreitertem Durchmesser ausgeführt. Endseitig weist der Fräser 6 mehrere Schneiden auf, und zwar Hauptschneiden 602 sowie Vorschneiden 603. Die Hauptschneiden 602, deren Position in Fig. 9 durch Balken 608 kenntlich gemacht ist, reichen längenmäßig über eine gesamte Wandstärke, nämlich vom Außendurchmesser des Fräsers 6 bis an eine lichte Weite des Fräskopfes 601. Die Hauptschneiden 602 liegen in einem Winkel von 90° zur kreisförmigen Schnittbewegung, sodass sich eine Schnittkraft gleichmäßig über eine Schneidenlänge der Hauptschneiden 602 verteilt. Die Hauptschneiden 602 führen die primäre Spanungsarbeit aus. Darüber hinaus sorgen die Hauptschneiden 602 für eine nötige Freistellung in der Schnittfuge und übernehmen das von den Vorschneiden 603 verdrängte Knochengewebe.

An den Vorschneiden 603, deren Position in Fig. 10 durch Balken 609 kenntlich gemacht ist, findet eine detaillierte Formgebung eines Knochen- und/oder Knorpelzylinders statt. Infolgedessen ist die von den Vorschneiden 603 verrichtete Spanungsarbeit im Gegensatz zur Spanungsarbeit der Hauptschneiden 602 sehr gering. Die Vorschneiden 603 überragen in Vorschubrichtung des Fräsers 6 die Hauptschneiden 602 und liegen am Innendurchmesser des Fräskopfes 601. Der schneidende Charakter der Vorschneiden 603 wird durch deren zirkuläre Position am Fräskopf 601 verdeutlicht. Ein initialer Kontakt mit einer Knochenoberfläche findet nur an den Vorschneiden 603 statt. Dabei führen die Vorschneiden 603 durch die entsprechende Anordnung den Fräser 6 beim Anschnitt der Kortikalis. Noch bevor die Hauptschneiden 602 eine gesamte Stärke beispielsweise eines Beckenknochens durchsetzt haben, lösen die um etwa 0,5 mm vorragenden Vorschneiden 603 den Knochenzylinder aus dem Gewebeverband. Ausschließlich Zähne mit den Vorschneiden 603 durchsetzen einen Beckenkamm zur Gänze.

Grundsätzlich sind jeweils drei Hauptschneiden 602 in abwechselnder Anordnung mit drei Vorschneiden 603 vorgesehen. Gleichwohl ist es auch möglich, eine höhere Anzahl von Hauptschneiden 602 sowie Vorschneiden 603 vorzusehen. Günstig ist es in jedem Fall, dass die Vorschneiden 603 die Hauptschneiden 602 nicht nur überragen, sondern auch einen kleineren Spitzenwinkel aufweisen, wie es in Fig. 11 angedeutet ist. Aufgrund einer Längendifferenz und eines kleineren Spitzenwinkels ermöglichen die Vorschneiden 603 einen verlaufsfreien Anschnitt einer Knochenoberfläche. Darüber hinaus sorgt eine Überlänge der Vorschneiden 603 in weiterer Folge zusammen mit dem Arbeitskanal 5, in dem der Fräser 6 geführt ist, für eine exakte Führung im Knochengewebe.

Der Fräskopf 601 ist wie erwähnt konisch ausgebildet und verjüngt sich von den Hauptschneiden 602 und den Vorschneiden 603 ausgehend zum Fräserschaft hin. Beispielsweise kann vorgesehen sein, dass sich der Fräserschaft von einem Außendurchmesser von 11 mm auf 9,5 mm im Bereich des Fräserschaftes verjüngt. Diese Verjüngung wird als Hinterschliff bezeichnet und bewirkt eine Freistellung des Fräsers 6 in einer Schnittfuge. Dasselbe Prinzip findet auch im Kern des Fräsers 6 seine Anwendung. Eine lichte Weite hingegen vergrößert sich von den Vorschneiden 603 in Richtung Fräserschaft. Durch diesen Hinterschliff kommt die Kronenform des Fräskopfes 601 zustande, welcher eine Freistellung des Fräsers 6 im Knochengewebe bewirkt. Am Umfang wird dadurch ein Reiben des Fräserschaftes am Knochen und eine damit verbundene Wärmeentwicklung verhindert. Gleichermaßen sorgt der Hinterschliff für die nötige Bewegungsfreiheit im Knochenkanal. Ein ausgefräster Knochenzylinder hat beispielsweise einen Durchmesser von 7,1 mm und wandert während eines Bearbeitungsvorganges in den Fräserschaft. Das entsprechende Biopsat kann ohne zusätzliche Reibung und Druckbelastung geborgen werden. Die spezielle Kronenform des Fräskopfes 601 verhindert durch Reduktion von Kontaktflächen zwischen Werkzeug und Bearbeitungswerkstoff eine übermäßige Wärmeentwicklung. Ein Kontakt sollte im Idealfall nur bei der spanenden Bearbeitung an den Vorschneiden 603 und den Hauptschneiden 602 des Fräsers 6 entstehen.

Eine Spanabfuhr erfolgt über Spannuten 604, die am äußeren Umfang des Fräsers 6 schräg vor jeder der drei Hauptschneiden 602 in Schnittrichtung angeordnet sind. Ein flüssiger Abtransport von Spänen führt zur Reduktion einer Wärmeentwicklung und beeinflusst eine Oberflächenqualität eines entnommenen Knochen- und/oder Knorpelzylinders positiv. Bei einem Bearbeitungsvorgang wird ein Knochenspan aus dem Gewebeverband gelöst und gleitet während des Vorganges über Spanflächen der Schneiden. Die Spannuten 604 beginnen an der Basis der jeweiligen Hauptschneide 602, von denen das abgesetzte Knochengewebe aufgenommen wird. Die gebildeten Späne werden in der Spannut gespeichert und über deren Verlauf in Richtung Fräserschaft aus der Schnittfuge abgeleitet. Ohne eine optimale Spanabfuhr würde sich abgetragenes Knochenmehl zwischen den einzelnen Schneiden sammeln und eine weitere Spanabnahme behindern, und zwar letztlich so lange, bis diese zum Stillstand käme. Der Fräser 6 würde sich dann unter steigender Wärmeentwicklung in der Schnittfuge reiben.

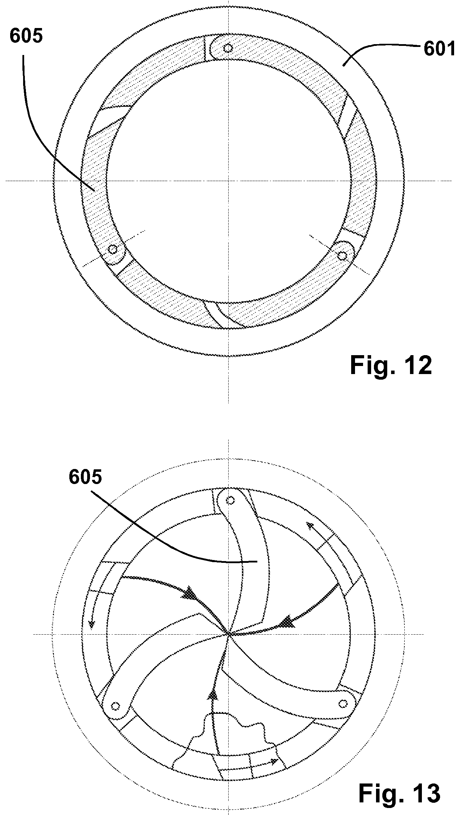

Bevorzugt weist der Fräser 6 nicht nur die erwähnten Hauptschneiden 602 und Vorschneiden 603 auf, sondern alternativ oder bevorzugt zusätzlich axial wirkende Schneiden 605. Diese zusätzlichen Schneiden 605 erweisen sich als günstig, wenn ein Knochen nicht vollständig durchfräst werden soll, sondern ein Biopsat oder Transplantat aus dem Knochen abgesetzt werden soll. Die zusätzlichen Schneiden 605, die in Fig. 12 und 13 dargestellt sind, sind im Fräskopf 601 unter den Hauptschneiden 602 bzw. Vorschneiden 603 angeordnet. Während einer Vorschubbewegung des Fräsers 6 befinden sich die zusätzlichen Schneiden 605 in einer inaktiven Position, wie dies in Fig. 12 dargestellt ist. Ist der Fräser 6 bis zu einer vorbestimmten Tiefe in das Knochengewebe eingedrungen und soll dort ein Biopsat oder allgemein Knochen- und/oder Knorpelstück abgesetzt werden, so werden die zusätzlichen Schneiden 605 aktiviert und schneiden quer zur Längsachse des Fräsers 6, wie dies in Fig. 13 dargestellt ist. Dadurch kann auf einfache Weise ein Knochenzylinder abgesetzt werden, ohne dass ein Knochen vollständig zu durchfräsen ist.

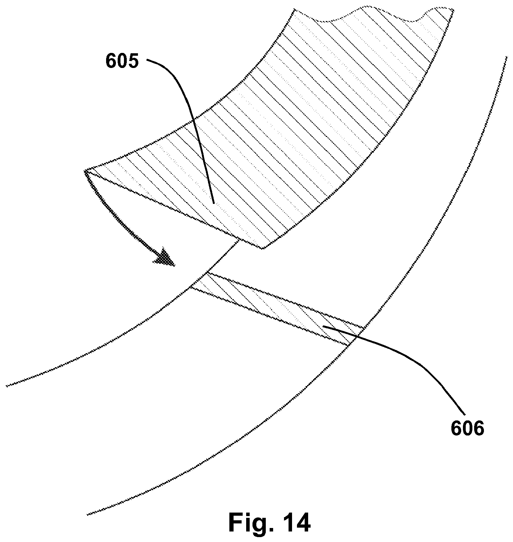

Eine Aktivierung der zusätzlichen Schneiden 605 kann auf verschiedene Arten erfolgen. Beispielsweise ist es wie in Fig. 14 schematisch dargestellt möglich, dass die zusätzlichen Schneiden 605 jeweils mit einem Magneten 606 in einer inaktiven, äußeren Position gehalten werden und dann bei Bedarf durch Deaktivierung der magnetischen Sicherung freigegeben werden können. Auf analoge Weise können die zusätzlichen Schneiden 605 durch Aktivierung der magnetischen Sicherung wieder in eine inaktive Position gebracht werden.

Weist der Fräser 6 nicht nur die erwähnten Hauptschneiden 602 und Vorschneiden 603 auf, sondern auch die zusätzlichen Schneiden 605, kann ein Knochen- und/oder Knorpelzylinder noch im Knochen abgesetzt werden, ohne dass eine gesamte Knochenstärke durchfräst werden muss. Die zusätzlichen Schneiden 605, vorzugsweise drei, sitzen in einer 90°-Position zur Längsachse des Fräsers 6, vorzugsweise direkt an einer Basis der Hauptschneiden 602 und der Vorschneiden 603. Neben der beschriebenen Ausführungsvariante können die zusätzlichen Schneiden 605 durch Passstifte in Position gehalten sein, um welche die zusätzlichen Schneiden 605 schwenkbar gelagert sind. Die zusätzlichen Schneiden 605 weisen bevorzugt eine sichelförmige Gestalt auf. Eine zusätzliche Spannut, die nicht dargestellt ist, zieht über eine ebene, an der Basis der Hauptschneiden 602 und Vorschneiden 603 grenzende Fläche jeder einzelnen Axialschneide. Dadurch können die abgetragenen Späne hinter die jeweilige Axialschneide effizient abgeführt werden, ohne die dahinterliegende Schneide bei einer Spanabnahme zu behindern. Während einer Vorschubbewegung des Fräsers 6 befinden sich die zusätzlichen Schneiden 605 in einer inaktiven Position.

Ist der Fräser 6 bis zu einer vorbestimmten Tiefe in das Knochengewebe eingefräst, kann ein Knochen- und/oder Knorpelstück durch Aktivierung der zusätzlichen Schneiden 605 wie erwähnt abgesetzt werden. Diesbezüglich können die zusätzlichen Schneiden 605 wie beschrieben aktiviert werden. Möglich ist es auch, dass die zusätzlichen Schneiden 605 durch mechanische Elemente aktiviert und deaktiviert werden können. Beispielsweise kann ein Mechanismus mit einer Zahnkrone mit keilförmigen Zähnen vorgesehen sein, wobei die Zahnkrone drehbar ist und wobei die Zähne bei Drehung der Zahnkrone gegen den Uhrzeigersinn die zusätzlichen Schneiden 605 aktivieren. Eine Deaktivierung kann durch eine Nut-Zapfen-Verbindung erreicht werden, welche die zusätzlichen Schneiden 605 mit den Zähnen der Zahnkrone jeweils an deren Rückseite verbindet. Dadurch können die zusätzlichen Schneiden 605 auf einfache Weise in eine inaktive Position gebracht werden.

### Eprouvette

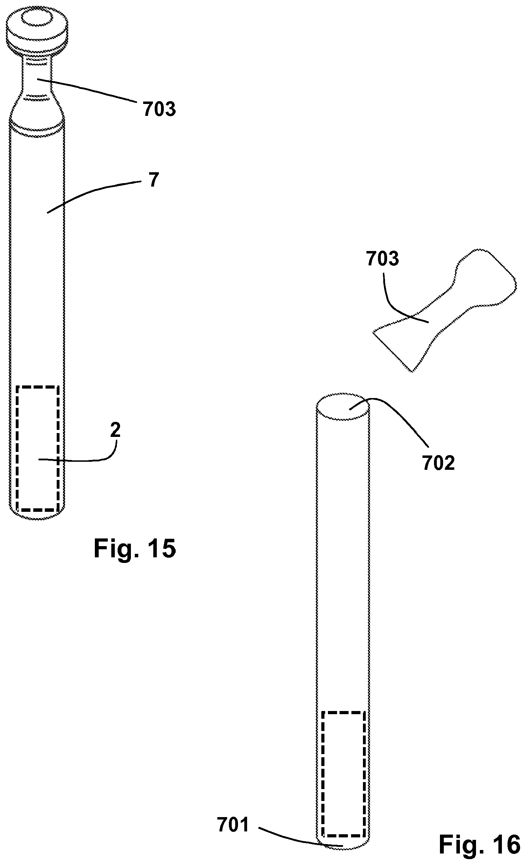

In Fig. 15 und 16 ist eine Eprouvette 7 dargestellt, die im hohlen Fräser 6 positioniert sein kann und zur Aufnahme eines Knochenbiopsats 2 dient. Die Eprouvette 7, die in der Regel aus einem Kunststoff wie Polyethylen gebildet ist, ist im Wesentlichen zylindrisch ausgebildet und weist einen äußeren Durchmesser auf, der zum inneren Durchmesser des Fräsers 6 im Bereich des Fräserschaftes korrespondiert. Die Eprouvette 7 ist in der Regel relativ zum Fräser 6 fixiert und in diesem innen anliegend angeordnet. Die Eprouvette 7 ist dabei im Bereich eines ersten Endes 701 durch eine nicht dargestellte Membran, die einen Schlitz aufweist, verschlossen. Dieses erste Ende 701 wird benachbart zum Fräskopf 601 im Fräser 6 positioniert. Im Bereich eines gegenüberliegenden zweiten Endes 702 ist die Eprouvette 7 durch ein Griffteil 703 verschlossen. Das Griffteil 703 kann von der Eprouvette 7 abgebrochen werden. Während eines Biopsievorganges dient die Eprouvette 7 zur Sicherung z. B. eines Knochenbiopsats 2. Nach Abschluss des Biopsievorganges befindet sich das Knochenbiopsat 2 in der Eprouvette 7 und kann dann bei Bedarf durch Abbrechen des Griffteils 703 auf einfache Weise entnommen werden.

### Kombiniertes Modul

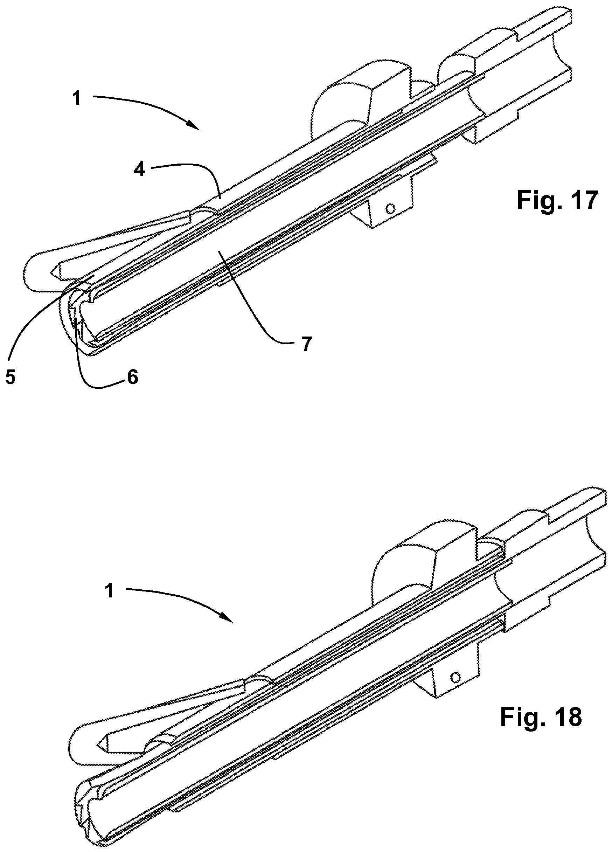

In Fig. 17 und 18 ist eine Kombination des Gewebekanals 4, des Arbeitskanals 5, des Fräsers 6 und der Eprouvette 7 dargestellt. Wie aus Fig. 17 ersichtlich ist, ist der Gewebekanal 4 außenseitig angeordnet. Im Gewebekanal 4 befindet sich der im Bereich des Grundkörpers 402 des Gewebekanals 4 anliegende Arbeitskanal 5. In der in Fig. 17 dargestellten Position ist der Arbeitskanal 5 bereits so weit in den Gewebekanal 4 eingeführt, dass dessen Klappen 403 sich in gespreizter Stellung befinden. Innerhalb des Arbeitskanals 5 wiederum ist der Fräser 6 angeordnet, wobei der Arbeitskanal 5 einen freien inneren Durchmesser aufweist, der einem äußeren Durchmesser des Fräskopfes 601 des Fräsers 6 entspricht. Da der Fräskopf 601 wie erwähnt konisch verbreitert ausgeführt ist, ist im Bereich des Fräserschaftes zwischen dem Arbeitskanal 5 und dem Fräser 6 ein Spalt gebildet, in welchen Späne aufgenommen werden können, die über die erläuterten Spannuten 604 in diesen Spalt befördert werden. Innerhalb des Fräsers 6 ist innen anliegend die erläuterte Eprouvette 7 angeordnet. Während eines Bearbeitungsvorganges bzw. eines Überganges von einer ersten Arbeitsposition gemäß Fig. 17 in eine zweite Arbeitsposition gemäß Fig. 18 sind der Gewebekanal 4 und der Arbeitskanal 5 im Gewebe fixiert gehalten und bleiben lagestabil. Der Fräser 6 mit der innen anliegenden Eprouvette 7 hingegen wird in das Knochengewebe vorgeschoben, um ein Knochen- und/oder Knorpelstück bzw. ein Knochenbiopsat 2 zu entnehmen. Dabei wandert das zu entnehmende Knochen- und/oder Knorpelstück in die innen anliegende Eprouvette 7 und wird vorerst durch die vorgesehene Membran, die einen Schlitz aufweist, gehalten, ehe das Knochen- und/oder Knorpelstück am Ende des Bearbeitungsvorganges letztlich nahezu vollständig in der Eprouvette 7 liegt. Nach Abschluss des Bearbeitungsvorganges kann der Fräser 6 mit der innen anliegenden Eprouvette 7 aus dem Arbeitskanal 5 gezogen werden. Dabei erweist sich die konisch verbreiterte Form des Fräskopfes 601 als günstig, weil dadurch die im Spalt zwischen dem Fräser 6 und dem Arbeitskanal 5 befindlichen Späne mit herausgezogen werden. Danach kann die Eprouvette 7 aus dem Fräser 6 entfernt werden und das Knochen- und/oder Knorpelstück aus der Eprouvette 7 entnommen oder gegebenenfalls auch in dieser für längere Zeit gelagert werden.

### Positioniervorrichtung

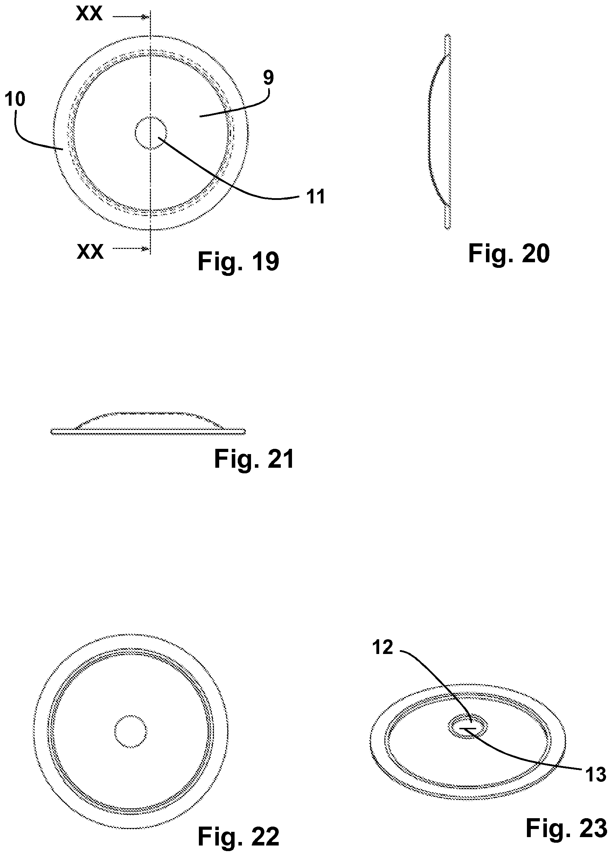

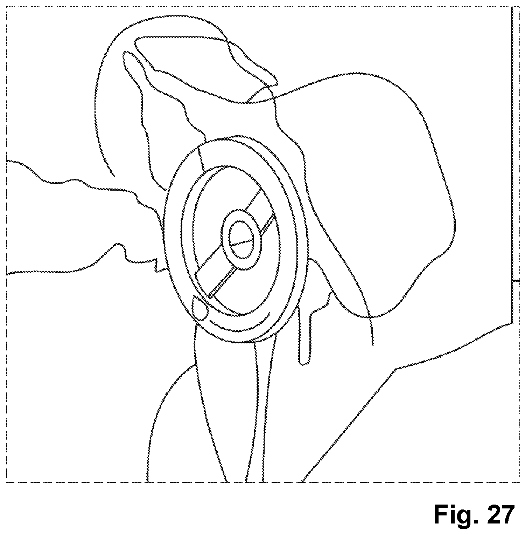

Zur Positionierung der zuvor erläuterten Komponenten erweist es sich als zweckmäßig, eine damit zusammenwirkende Einrichtung 8 vorzusehen, deren Aufbau und Wirkungsweise anhand von Fig. 19 bis 27 erläutert ist. Die Einrichtung 8 umfasst eine Druckplatte 9 mit einem verstärkten Rahmen 10. Im Zentrum der Druckplatte 9 ist eine kreisförmige Öffnung 11 vorgesehen. Die Öffnung 11 weist einen Durchmesser auf, der in etwa einem äußeren Durchmesser des Gewebekanals 4 im Bereich des Grundkörpers 402 entspricht. In der Öffnung 11 ist eine Membran 12 positioniert, die eine balkenförmige Markierung 13 aufweist.

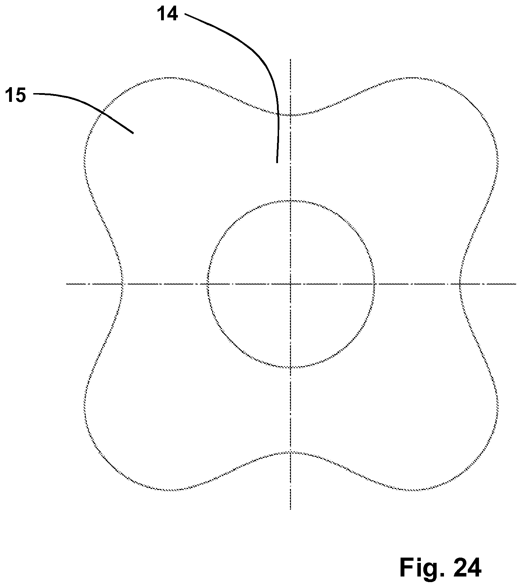

Die Druckplatte 9 ist aus einem Kunststoff wie beispielsweise Polypropylen gefertigt, insbesondere mittels eines Spritzgussverfahrens. Der Rahmen 10 ist wie erwähnt verstärkt ausgebildet. In den übrigen Bereichen bzw. zum Zentrum hin besteht die Druckplatte 9 aus einem dünnen Plättchen, das durch händischen Druck zwischen zwei konkaven Stellungen hin und her gedrückt werden kann. An den Rahmen 10 schließt eine in Fig. 24 dargestellte Haftfolie 14 an. In der in Fig. 24 ersichtlichen zentralen Ausnehmung der Haftfolie 14 wird bzw. ist die Druckplatte 9 positioniert, und zwar so, dass die Haftfolie 14 am Rahmen 10 angreift. Zweckmäßig ist es, wie in Fig. 24 dargestellt, dass die Haftfolie 14 mehrere Laschen 15 aufweist, sodass die wenige zehntel Millimeter starke Haftfolie 14 gut haftend auf verschiedenen Positionen eines Körpers angeordnet werden kann. Dabei sind entlang einer Verbindung zwischen der Haftfolie 14 und dem Rahmen 10 Sollbruchstellen vorgesehen, sodass die Haftfolie 14 später vom Rahmen 10 entfernt werden kann.

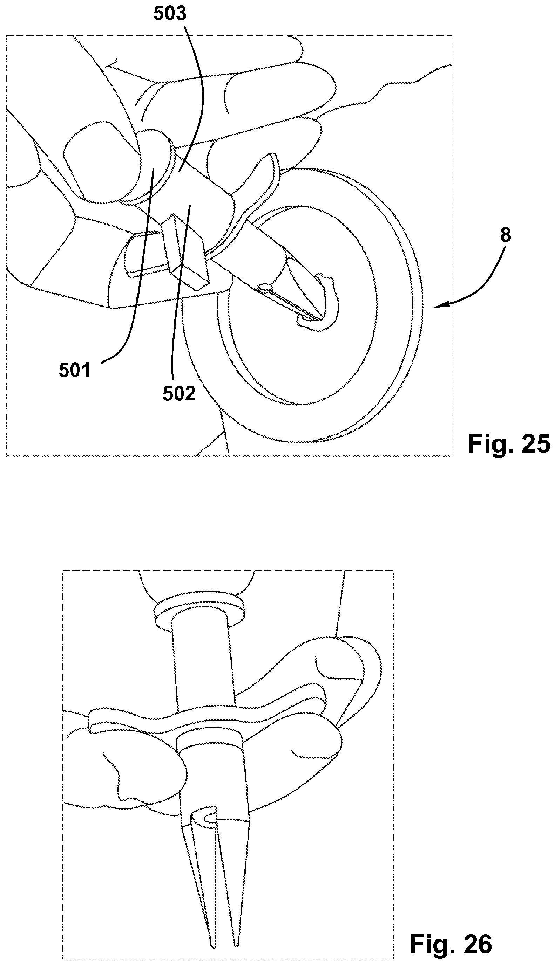

Im Einsatz bzw. bei einem Bearbeitungsvorgang wird die Haftfolie 14 mit der verbundenen Druckplatte 9 auf einen Körper aufgeklebt. Anschließend wird die Druckplatte 9 durch leichten händischen Druck in eine konkave Stellung gebracht bzw. umgewölbt, sodass die Druckplatte 9 unter Druck am Körper anliegt. Die Haftfolie 14 und die Druckplatte 9 sind nun fest am Körper befestigt. Die Haftfolie 14 stützt das Gewebe rund um ein Biopsieareal, dient aber primär als prä- und perioperative Keimbarriere. In der Mitte der Druckplatte 9 bzw. im Bereich der Membran 12 liegt das sogenannte Biopsiefenster. In dieses Biopsiefenster kann durch Einbringung eines kurzen Gewebeschnittes durch die Schneidelemente der Gewebekanal 4 samt dem Arbeitskanal 5 eingeführt werden, wie dies in Fig. 25 dargestellt ist. Während dieser Phase wird der Arbeitskanal 5 durch das Distanzstück 502 in einer Position gehalten, in welcher die Klappen 403 des Gewebekanals 4 nicht geöffnet werden können. Diesbezüglich ist es zweckmäßig, dass der Arbeitskanal 5 endseitig einen abnehmbaren Stempel 501 aufweist. Ist der Gewebekanal 4 samt innen anliegendem Arbeitskanal 5 eingeführt, so kann das Distanzstück 502 entfernt werden und der Arbeitskanal 5 in den Gewebekanal 4 zum Öffnen der Klappen 403 vorangetrieben werden. Dies ist in Fig. 25 dargestellt, wobei das Gewebe nicht gezeigt ist. Sind die Klappen 403 im Gewebe aufgespreizt, so ist der Gewebekanal 4 fixiert. Der Arbeitskanal 5 kann dann weiter vorgetrieben werden, bis dessen endseitige Zacken 504 ebenfalls verankert sind. Gleichzeitig wird das Griffstück 406 des Gewebekanals 4 in einer Nut des Rahmens 10 der Druckplatte 9 fixiert. Zusätzlich oder alternativ kann der Gewebekanal 4 auch mit dessen Sicherungsrasten 410 an einem verdickten inneren Rand der Öffnung 11 der Druckplatte 9 gegebenenfalls geneigt einrasten, wie dies in Fig. 2 angedeutet ist. Es kann nun der Fräser 6 samt innen anliegender Eprouvette 7 in den Arbeitskanal 5 eingeführt werden, um ein Knochen- und/oder Knorpelstück wie ein Knochenbiopsat 2 zu entnehmen. Ist das Knochen- und/oder Knorpelstück entnommen, so wird der Fräser 6 wie bereits erläutert aus dem Arbeitskanal 5 gezogen. Anschließend kann der Gewebekanal 4 samt Arbeitskanal 5 durch Lösen des Griffstücks 406 aus der Nut des Rahmens 10 und Herausziehen gelöst werden. Die Einrichtung 8 dient nun in einer zweiten Funktion zur Wundversorgung. In einem ersten Schritt wird die Haftfolie 14, die als Keimbarriere dient, entlang der Sollbruchstellen vom Rahmen 10 gelöst. Die Druckplatte 9 samt Rahmen 10 kann noch länger zwecks Blutstillung am Körper anliegen. Anschließend wird auch die Druckplatte 9 abgenommen, wobei lediglich die zentrale Membran 12, die mit der Druckplatte 9 ebenfalls über Sollbruchstellen verbunden ist, am Körper zurückbleibt. Ist schließlich die kleine Wunde verheilt, kann auch die Membran 12 entfernt werden. Die Einrichtung 8 hat somit den Vorteil, dass diese sowohl zur Positionierung von Komponenten zur Entnahme eines Knochen- und/oder Knorpelstücks als auch zur nachfolgenden Wundversorgung dient.

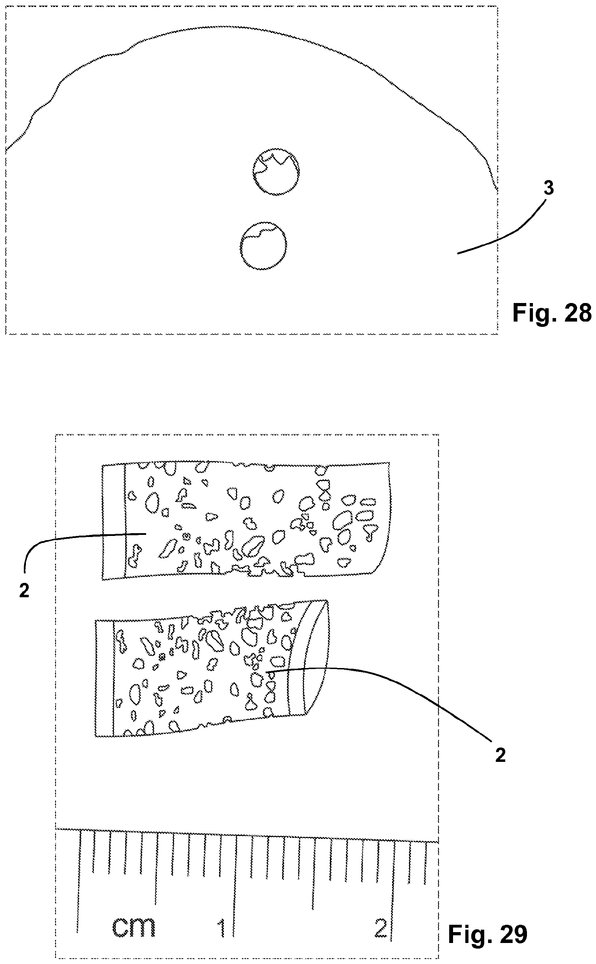

In Fig. 28 ist ein Beckenknochenteil 3 dargestellt, aus dem zwei Knochenbiopsate 2 entnommen wurden, die in Fig. 29 dargestellt sind. Wie ersichtlich ist, sind die entnommenen Knochenbiopsate 2 völlig intakt und können für nachfolgende Untersuchungen genutzt werden. Sinngemäßes trifft für Knochen- und/oder Knorpelstücke zu, die zu Transplantationszwecken entnommen werden.

## Patentansprüche

1. Vorrichtung (1) zum Entnehmen eines Knochen- und/oder Knorpelstücks am knöchernen Skelett eines Menschen oder Tieres, insbesondere eines Knochenzylinders, beispielsweise eines Beckenknochenteils (3) eines Menschen, wobei ein hohler Gewebekanal (4) und ein hohler Fräser (6) mit endseitigen Schneiden vorgesehen sind, wobei der Fräser (6) im Inneren des Gewebekanals (4) geführt werden kann, **dadurch gekennzeichnet, dass** der Gewebekanal (4) endseitig durch einen zulaufenden Konus (401) bildende Klappen (403) verschlossen ist.

2. Vorrichtung (1) nach Anspruch 1, **dadurch gekennzeichnet, dass** ein hohler Arbeitskanal (5) vorgesehen ist, der im Gewebekanal (4) innen anliegend angeordnet ist und mit dem durch Vorschub die Klappen (403) öffenbar sind, wobei der Fräser (6) im Arbeitskanal (5) geführt ist.

3. Vorrichtung (1) nach Anspruch 1 oder 2, **dadurch gekennzeichnet, dass** der Gewebekanal (4) aus Kunststoff besteht und insbesondere einteilig ausgebildet ist.

4. Vorrichtung (1) nach einem der Ansprüche 1 bis 3, **dadurch gekennzeichnet, dass** zwei Klappen (403) vorgesehen sind.

5. Vorrichtung (1) nach einem der Ansprüche 1 bis 4, **dadurch gekennzeichnet, dass** die Klappen (403) an deren Enden zumindest teilweise, vorzugsweise jeweils, ein oder mehrere Schneidelemente (404) wie Metallklingen aufweisen.

6. Vorrichtung (1) nach einem der Ansprüche 1 bis 5, **dadurch gekennzeichnet, dass** die Klappen (403) vor dem Öffnen über Sollbruchstellen (405) miteinander verbunden sind.

7. Vorrichtung (1) nach einem der Ansprüche 1 bis 6, **dadurch gekennzeichnet, dass** der Gewebekanal (4) mit einem endseitigen Griffstück (406) ausgebildet ist und der Arbeitskanal (5) einen endseitig lösbar befestigten Stempel (501) aufweist, der in Vorschubrichtung nicht über das Griffstück (406) hinausbewegbar ist.

8. Vorrichtung (1) nach einem der Ansprüche 1 bis 7, **dadurch gekennzeichnet, dass** der Fräser (6) einen endseitig in Vorschubrichtung im Durchmesser verbreiterten Fräskopf (601) mit abwechselnd angeordneten Hauptschneiden (602) und Vorschneiden (603) aufweist.

9. Vorrichtung (1) nach einem der Ansprüche 1 bis 8, **dadurch gekennzeichnet, dass** im Fräser (6) eine innen anliegende hohle Eprouvette (7) positioniert ist.

10. Vorrichtung (1) nach Anspruch 9, **dadurch gekennzeichnet, dass** die Eprouvette (7) relativ zum Fräser (6) fixiert ist.

11. Vorrichtung (1) nach Anspruch 9 oder 10, **dadurch gekennzeichnet, dass** die Eprouvette (7) im Bereich eines ersten Endes (701) durch eine Membran mit einem Schlitz verschlossen ist.

12. Vorrichtung (1) nach einem der Ansprüche 9 bis 11, **dadurch gekennzeichnet, dass** die Eprouvette (7) im Bereich eines zweiten Endes (702) durch ein Griffteil (703) verschlossen ist, wobei Sollbruchstellen vorgesehen sind, sodass das Griffteil (703) von der Eprouvette (7) abbrechbar ist.

13. Vorrichtung (1) nach einem der Ansprüche 1 bis 12, **dadurch gekennzeichnet, dass** der Fräser (6) mit einem hohlen Schaft ausgebildet ist und endseitig einen Fräskopf (601) mit abwechselnd angeordneten Hauptschneiden (602) und Vorschneiden (603) aufweist, wobei die Vorschneiden (603) die Hauptschneiden (602) in axialer Richtung überragen, und/oder am Fräser (6) neben vertikal schneidenden Schneiden zusätzliche Schneiden (605) vorgesehen sind, die normal zu einer Längsachse des Fräsers (6) schneiden und bei Bedarf aktivierbar sind.

14. Vorrichtung (1) nach Anspruch 13, **dadurch gekennzeichnet, dass** die Vorschneiden (603) einen kleineren Spitzenwinkel aufweisen als die Hauptschneiden (602).

## Claims

1. A device (1) for removing a bone- and/or cartilage section on the osseous skeleton of a human or animal, in particular for removing a bone cylinder, for example a part of the pelvic bone (3) of a human, wherein a hollow tissue channel (4) and a hollow cutter (6) with blades on the end side are provided, wherein the cutter (6) can be guided in the interior of the tissue channel (4), **characterized in that** the tissue channel (4) is closed on the end side by flaps (403) forming a tapered cone (401).

2. The device (1) according to Claim 1, **characterized in that** a hollow working channel (5) is provided, which is arranged fitting internally in the tissue channel (4) and by which the flaps (403) are able to be opened by an advance motion, wherein the cutter (6) is guided in the working channel (5).

3. The device (1) according to Claim 1 or 2, **characterized in that** the tissue channel (4) consists of plastic and in particular is formed in one piece.

4. The device (1) according to one of Claims 1 to 3, **characterized in that** two flaps (403) are provided.

5. The device (1) according to one of Claims 1 to 4, **characterized in that** the flaps (403) have at their ends at least partially, preferably respectively, one or more cutting elements (404) such as metal blades.

6. The device (1) according to one of Claims 1 to 5, **characterized in that**, before opening, the flaps (43) are connected with one another via predetermined breaking points (405).

7. The device (1) according to one of Claims 1 to 6, **characterized in that** the tissue channel (4) is formed with handle piece (406) on the end side, and the working channel (5) has a punch (501), detachably fastened on the end side, which is not able to be moved beyond the handle piece (406) in the feed direction.

8. The device (1) according to one of Claims 1 to 7, **characterized in that** the cutter (6) has a cutting head (601), widened in diameter at the end side in the feed direction, with alternately arranged main blades (602) and preliminary blades (603).

9. The device (1) according to one of Claims 1 to 8, **characterized in that** an internally fitting hollow specimen tube (7) is positioned in the cutter (6).

10. The device (1) according to Claim 9, **characterized in that** the specimen tube (7) is fixed relative to the cutter (6).

11. The device (1) according to Claim 9 or 10, **characterized in that** the specimen tube (7) is closed in the region of a first end (701) by a membrane with a slot.

12. The device (1) according to one of Claims 9 to 11, **characterized in that** the specimen tube (7) is closed in the region of a second end (702) by a grip part (703), wherein predetermined breaking points are provided, so that the grip part (703) is able to be broken off from the specimen tube (7).

13. The device (1) according to one of Claims 1 to 12, **characterized in that** the cutter (6) is formed with a hollow shaft and has at the end side a cutting head (601) with alternately arranged main blades (602) and preliminary blades (603), wherein the preliminary blades (603) protrude beyond the main blades (602) in axial direction, and/or in addition to vertically cutting blades, additional blades (605) are provided on the cutter (6), which cut perpendicularly to a longitudinal axis of the cutter (6) and are able to be activated when required.

14. The device (1) according to Claim 13, **characterized in that** the preliminary blades (603) have a smaller point angle than the main blades (602).

## Revendications

1. Dispositif (1) pour retirer une pièce osseuse et/ou de cartilage sur le squelette osseux d'un humain ou d'un animal, en particulier un cylindre osseux, par exemple une pièce osseuse du bassin (3) d'un humain, dans lequel un canal à tissu creux (4) et une fraise creuse (6) avec des lames à l'extrémité sont prévus, dans lequel la fraise (6) peut être dirigée à l'intérieur du canal à tissu (4), **caractérisé en ce que** le canal à tissu (4) est fermé à l'extrémité par un volet (403) formant un cône (401) se terminant en pointe.

2. Dispositif (1) selon la revendication 1, **caractérisé en ce qu'**un canal de travail creux (5) est prévu, qui est disposé ajusté à l'intérieur du canal à tissu (4) et avec lequel les volets (403) peuvent être ouverts par l'avancement, dans lequel la fraise (6) est dirigée dans le canal de travail (5).

3. Dispositif (1) selon la revendication 1 ou 2, **caractérisé en ce que** le canal à tissu (4) est en plastique et est conçu en particulier d'une seule pièce.

4. Dispositif (1) selon l'une des revendications 1 à 3, **caractérisé en ce que** deux volets (403) sont prévus.

5. Dispositif (1) selon l'une des revendications 1 à 4, **caractérisé en ce que** les volets (403) présentent sur les extrémités, au moins partiellement, de préférence respectivement, un ou plusieurs éléments de coupe (404) comme des lames métalliques.

6. Dispositif (1) selon l'une des revendications 1 à 5, **caractérisé en ce que** les volets (403) sont reliés entre eux par des points de rupture (405) devant l'ouverture.

7. Dispositif (1) selon l'une des revendications 1 à 6, **caractérisé en ce que** le canal à tissu (4) est conçu avec une partie de poignée (406) à l'extrémité et le canal de travail (5) présente un poinçon (501) pouvant être fixé de manière séparable à l'extrémité qui ne peut pas être déplacé au-delà de la partie de poignée (406) dans le sens d'avancement.

8. Dispositif (1) selon l'une des revendications 1 à 7, **caractérisé en ce que** la fraise (6) présente une tête de fraisage (601) élargie en diamètre à l'extrémité dans le sens d'avancement avec des lames principales (602) et des lames avant (603) disposées en alternance.

9. Dispositif (1) selon l'une des revendications 1 à 8, **caractérisé en ce qu'**une éprouvette (7) creuse est positionnée ajustée à l'intérieur de la fraise (6).

10. Dispositif (1) selon la revendication 9, **caractérisé en ce que** l'éprouvette (7) est fixe par rapport à la fraise (6).

11. Dispositif (1) selon la revendication 9 ou 10, **caractérisé en ce que** l'éprouvette (7) est fermée par une membrane à fente au niveau de sa première extrémité (701).

12. Dispositif (1) selon l'une des revendications 9 à 11, **caractérisé en ce que** l'éprouvette (7) est fermée par une partie de poignée (703) au niveau de sa seconde extrémité (702), dans lequel des points de rupture sont prévus, de sorte que la partie de poignée (703) peut être rompue par l'éprouvette (7).

13. Dispositif (1) selon l'une des revendications 1 à 12, **caractérisé en ce que** la fraise (6) est conçue avec une tige creuse et présente à l'extrémité une tête de fraisage (601) avec des lames principales (602) et des lames avant (603) disposées en alternance, dans lequel les lames avant (603) dépassent des lames principales (602) dans le sens axial et/ou des lames (605) supplémentaires sont prévues sur la fraise (6) près de lames coupant verticalement, qui coupent en normale par rapport à un axe longitudinal de la fraise (6) et peuvent être activées si besoin.

14. Dispositif (1) selon la revendication 13, **caractérisé en ce que** les lames avant (603) présentent un angle au sommet plus petit que les lames principales (602).
